# EUROPEAN PATENT SPECIFICATION

(11) **EP 3 271 287 B1**
(45) Date of publication and mention of the grant of the patent: **06.05.2020**
(21) Application number: 16713180.4
(22) Date of filing: 07.03.2016
(51) Int. Cl.: B82Y 5/00, A61F 2/00, A61L 27/00, A61B 17/00

(54) **DEVICES AND KITS FOR ATTACHING SURGICAL MESHES TO TISSUE**
VORRICHTUNGEN UND KITS ZUR BEFESTIGUNG CHIRURGISCHE NETZE AN GEWEBE
DISPOSITIFS ET KITS SERVANT À FIXER DES TREILLIS CHIRURGICAUX À UN TISSU

(30) Priority: 16.03.2015 US 201562133613 P
(43) Date of publication of application: 24.01.2018
(73) Proprietor: Boston Scientific Scimed, Inc., Maple Grove, MN 55311-1566 (US)
(72) Inventor: ZOLL, Jonathan, Brookline, Massachusetts 02446 (US); PEREIRA, Peter J., Mendon, Massachusetts 01756 (US)
(74) Representative: Vossius & Partner Patentanwälte Rechtsanwälte mbB
(86) International application number: PCT/US2016/021235
(87) International publication number: WO 2016/148958

(56) References cited:
- WO-A1-2015/089020
- US-A1- 2002 028 980
- US-A1- 2003 093 092
- US-A1- 2013 190 245
- US-B1- 6 875 427

## Description

### FIELD

The present disclosure relates to devices for the attachment of surgical meshes to tissue.

### BACKGROUND

Pelvic floor disorders are highly prevalent among women and involve a dropping down (prolapse) of the bladder, rectum and/or uterus caused by weakness of or injury to the ligaments, connective tissue, and muscles of the pelvis. Pelvic floor disorders are commonly treated by implanting a surgical mesh within the patient's pelvis to support the organ or organs that require support,

Surgical meshes are also employed in various other soft tissue applications as well. Examples of further uses for surgically implantable meshes include meshes for hernia repair (e.g., meshes for inguinal hernia, hiatus hernia, etc.), meshes for treatment of stress urinary incontinence, meshes for thoracic wall defects, breast support meshes and various other soft-tissue surgical mesh support devices, including meshes for cosmetic and reconstructive surgery, among others.

WO 2015/089020 A1 describes methods, compositions, devices and kits which pertain to the attachment of surgical meshes to tissue by application of an energy source to the meshes and tissue in the presence of a bonding material.

US 2013/190245 A1 discloses a light activated collagen-flavin composite layer incorporating riboflavin as treatment for infected lesions caused by bacteria and as the consequence of surgical procedures. The riboflavin in the collagen layer is exposed to light having a wavelength, for example, between 360-375 nm or 440-480 nm, decomposing the riboflavin to form reactive oxygen species. Crosslinks between the collagen composite and tissue result.

US 2003/093092 A1 discloses localized induction of hyperthermia in tissue or materials by delivering nanoparticles to the tissue or materials and exposing the nanoparticles to an excitation source under conditions wherein they emit heat. The generation of heat effects the joining of the tissue or materials.

The present disclosure pertains to methods, compositions, devices and kits that are useful in the attachment of surgical meshes to tissue in conjunction with these and other procedures.

### SUMMARY

The present disclosure relates to a surgical mesh according to claim 1.

In accordance with some aspects, a surgical mesh is provided which comprises a mesh material and a bonding material associated with the mesh material, wherein the surgical mesh is configured to bond to patient tissue when exposed to an energy source while the surgical mesh is in contact with the tissue. Preferred energy sources include those that target the bonding material and pass through tissue without damaging it by operating within a wavelength that has minimal interaction with tissue, such as near-infrared (NIR) energy sources.

In this regard, a surgical mesh is provided that is configured to bond to patient tissue when exposed to a near-infrared energy source while the surgical mesh is in contact with tissue. The mesh comprises a mesh material and a tissue bonding material that is associated with the mesh material, and the tissue bonding material comprises a tissue solder and an absorber material.

According to the invention the absorber material is a near-infrared absorber material. For example the near-infrared absorber material may display an absorbance peak between 650 nm and 1870 nm where light has its maximum depth of penetration in tissue, more typically between 650 nm and 950 nm. The near-infrared absorber material comprises nanoparticles in some instances, which may be selected, for example, from near-infrared absorbing metallic nanoparticles, near-infrared absorbing semiconductor nanoparticles, near-infrared absorbing carbon nanoparticles and near-infrared absorbing dyes.

In some embodiments, the absorber material is a radiofrequency absorber material. The radiofrequency absorber material may comprise, for instance, particles, which may be selected from conductive particles, magnetic particles and carbon particles, among others.

In some embodiments, which may be used in combination with any of the above aspects and embodiments, the tissue solder may be selected from chitosan, albumin, collagen, cellulose, elastin, nano-peptides, derivatives of the foregoing, and combinations of two or more of the foregoing.

In accordance with other aspects of the present disclosure, which do not fall under the invention, a method of attaching a surgical mesh to patient tissue is provided which comprise placing a mesh material and a bonding material in contact with patient tissue and using energy from an energy source to apply energy to the bonding material, such that the bonding material is activated and the mesh material is attached to the tissue. Preferred energy sources include those that target the bonding material and pass through tissue without damaging it by operating within a wavelength that has minimal interaction with tissue, such as radiofrequency (RF) energy sources and near-infrared (NIR) energy sources. Preferred bonding materials include those that comprise an absorber material that has an absorption peak at such wavelengths.

In this regard, in various aspects, the present disclosure pertains to a method of attaching a surgical mesh to patient tissue that comprises (a) placing a surgical mesh in accordance with any of the above aspects and embodiments in contact with patient tissue, the surgical mesh being configured to bond to patient tissue when exposed to near-infrared energy or radiofrequency energy while the surgical mesh is in contact with the tissue and (b) using an energy source to apply near-infrared energy or radiofrequency energy to the surgical mesh, such that the bonding material is activated and the mesh material is attached to the tissue.

In some embodiments, energy from the energy source travels through intervening tissue prior to reaching the bonding material. In some embodiments, energy from the energy source travels at least 1 mm of intervening tissue prior to reaching the bonding material.

In some embodiments, which may be used in combination with any of the above aspects and embodiments, the mesh material and the bonding material may be placed in contact with an anterior vaginal wall, a posterior vaginal wall, or both, while the energy source may be positioned inside the vagina such that energy from the energy source travels through the vaginal wall prior to reaching the bonding material.

Other aspects of the present disclosure pertain to a vaginal irradiation system that comprises a vaginal manipulation device that comprises a proximal handle, an elongate shaft and a distal portion comprising one or more energy sources. The vaginal manipulation device may be configured (a) to be inserted into a vagina such that the distal portion positioned at a distal end of the vagina and (b) to expose a tissue bonding material on an outer surface of the vagina to energy from the one or more energy sources.

In some embodiments, the vaginal manipulation device comprises one or more energy emitting spikes that are configured to puncture vaginal wall tissue and expose the tissue bonding material on the outer surface of the vagina to energy from the one or more energy sources. In some embodiments, the one or more energy sources of the vaginal manipulation device are configured to expose the tissue bonding material on the outer surface of the vagina to energy from the one or more energy sources, without puncturing vaginal wall tissue.

In some embodiments, which may be used in combination with any of the above aspects and embodiments, the one or more energy sources comprise one or more radiofrequency energy sources or one or more NIR-emitting energy sources.

In some embodiments, which may be used in combination with any of the above aspects and embodiments, the one or more energy sources produce near-infrared (NIR) or radiofrequency (RF) energy to which vaginal wall tissue is substantially transparent.

In some embodiments, which may be used in combination with any of the above aspects and embodiments, the one or more energy sources comprise one or more NIR-emitting sources selected from NIR-emitting diodes, NIR-emitting lasers, NIR-emitting laser diodes and NIR-emitting optical fibers.

In some embodiments, which may be used in combination with any of the above aspects and embodiments, the vaginal manipulation device comprises (a) a main body comprising a lumen and (b) an elongated member having a distal portion that comprises the one or more energy sources, which is disposable in the lumen and moveable longitudinally within the lumen.

In some embodiments, which may be used in combination with any of the above aspects and embodiments, the vaginal irradiation system further comprises (a) one or more thermal sensors for monitoring an increase in temperature produced by the one or more energy sources and (b) a controller for adjusting the output of the one or more energy sources based on data from the one or more thermal sensors.

Still other aspects of the present disclosure pertain to a kit comprising any combination of any two or more of the following items: (a) a surgical mesh in accordance with any of the above aspects and embodiments, (b) a vaginal manipulator device in accordance with any of the above aspects and embodiments, and (c) a surgical instrument configured to hold and place a surgical mesh.

These and other aspects, as well as various embodiments and advantages of the present disclosure will become immediately apparent to those of ordinary skill in the art upon review of the Detailed Description and claims to follow.

### BRIEF DESCRIPTION OF THE DRAWINGS

Fig. 1 is a schematic illustration of surgical mesh that has been implanted in the course of a sacrocolpopexy procedure, in accordance with an embodiment of the present disclosure.
Fig. 2 is a schematic illustration of a surgical mesh, in accordance with an embodiment of the present disclosure.
Figs. 3A-3B are schematic illustrations showing a medical procedure for implanting surgical mesh, in accordance with an embodiment of the present disclosure.
Fig. 4 is a schematic illustration showing a device and method for implanting a surgical mesh, in accordance with an embodiment of the present disclosure.
Figs. 5-8 are schematic illustrations of meshes, in accordance with various additional embodiments of the present disclosure.
Figs. 9A-9D are illustrations of existing devices suitable for vaginal manipulation during a sacrocolpopexy procedure.

### DETAILED DESCRIPTION

A more complete understanding of the present disclosure is available by reference to the following detailed description of numerous aspects and embodiments of the disclosure. The detailed description which follows is intended to illustrate but not limit the disclosure.

In various beneficial embodiments, tissue bonding technology is used as a technique for attaching surgical meshes to tissue. This is achieved by placing a mesh material and a bonding material in intimate association with patient tissue and then applying energy from an energy source to activate the bonding material and bond the mesh to the tissue. In this way, the present disclosure provides for the attachment of surgical mesh to patient tissue without the need for sutures, staples or other mechanical fasteners. In some embodiments, the mesh material and bonding material may be applied separately to the tissue, whereas in other embodiments, the mesh material and bonding material may be combined when applied to tissue, for example, by providing a coating of a bonding material on all or a portion of a mesh material or by incorporating a bonding material into all or a portion of a mesh material, among other strategies.

Different energy sources may be used for mesh attachment, depending on the mechanism for tissue bonding that is employed. The energy source may be, for example, a source of heat or light, such as a laser, a light-emitting diode (LED), a radiofrequency source, or a locally produced plasma, among others.

Preferred energy sources for use in the present disclosure are those that target the bonding material and pass through tissue without damaging it, by operating within a wavelength that has minimal interaction with tissue, such as radiofrequency (RF) energy sources and near-infrared (NIR) energy sources.

One example an energy source having these characteristics is an energy source that operates in a near-infrared tissue window (also known as an optical window or a therapeutic window) and radiates light at wavelengths ranging from 650 nm to 1870 nm where light has its maximum depth of penetration in tissue. Various imaging techniques have focused on a first NIR tissue window at wavelengths ranging from 650 to 950 nm, a second NIR tissue window at wavelengths ranging from 1100 to 1350 nm, and a third NIR tissue window at wavelengths ranging from 1600 to 1870 nm, which windows are useful for devices and methods employed herein.

There are currently available a wide range of NIR light sources including NIR-emitting diodes, NIR-emitting lasers, NIR-emitting laser diodes, incandescent sources filtered for NIR, and supercontinuum lasers. For instance, Excelitas Technologies Corp., Waltham, MA, UAS offers NIR-emitting diodes (also referred to as NIR-emitting LEDs), which operate at wavelengths of 770 nm, 870 nm 880 nm, 940 nm, and 950 nm. Laser diodes emitting at 785 nm, 808 nm and 1310 nm are available from Laser Components GmbH, Olching, Germany.

Other examples of energy sources that radiate energy having wavelengths that achieve minimal interaction with (and thus substantial penetration into) tissue include those that operates in a radiofrequency (RF) frequency range, for example, within a range of 3 kHz to 300 GHz. Various RF applications for medical use are based on shortwave RF frequencies (13.56 MHz) as licensing agencies permit this frequency for medical use.

A variety of bonding materials can be used in conjunction with the present disclosure.

In this regard, laser tissue soldering processes are known in the surgical art whereby tissue is bonded by applying a solder (commonly, a biological polymer) to the tissue after which a laser is used to activate the solder and form a bond. Without wishing to be bound by theory, it has been reported that the mechanism of laser tissue soldering appears to include a heating-induced protein denaturation-renaturation process. See, e.g., B. Forer et al., Laryngoscope 116: June 2006, 1002-1006.

In some embodiments solder materials are used in the present disclosure as bonding materials to bond mesh materials to tissue surfaces, for example, by heating the solder material while it is in contact with the mesh material and a tissue surface, such that the mesh material is fastened at one or more desired locations on the tissue surface.

Particularly beneficial solder materials have a relatively low activation temperature, and are bio-absorbable. Over time (typically between about 4 and 30 days, depending on the solder that is used), the solder may be bioabsorbed, leaving only mesh and tissue growth behind.

Specific solder materials for use in conjunction with the present disclosure include solders of biological origin and synthetic solders. Examples of solders of biological origin include those based on biological polymers, for example, polypeptides including nano-peptides and proteins such as albumin, collagen, elastin, fibrinogen, and fibrin, protein derivatives, as well as polysaccharides including cellulose and chitosan, among others. In some embodiments, two, three, four or more solder materials such as just described are employed. Specific examples include a combination of albumin and collagen, a combination of albumin and chitosan, a combination of collagen and chitosan, and a combination of albumin, collagen, and chitosan, among many other possible combinations.

In addition to, or instead of, a solder material, in some embodiments, the bonding material contains a photosensitizing dye, for example, one selected from rose bengal dye, methylene blue dye, fluorescein dye, indocyanine green, basic fuchsin, fen, xanthane dye, riboflavin dye, lumichrome dye, flavin, lumiflavin dye, Reactive Black 5 dye, and combinations of two or more of the foregoing.

In this regard, photochemical (i.e., photon based) tissue bonding processes are known the surgical art. These processes take advantage of the photochemical reactions that occur at intimately associated tissue surfaces, which are stained with a photosensitizing dye (e.g., dyed tissue surfaces which are placed in contact with one another). Without wishing to be bound by theory, it is believed that the dye absorbs photons (e.g., visible photons, ultraviolet photons, NIR photons, etc.) and promotes the formation of covalent bonds by crosslinking protein on the approximated tissue surfaces. For example, reactive species that are produced upon photon activation of the dye can react with potential electron donors and acceptors such as amino acids in proteins (e.g., tryptophan, tyrosine, cysteine, and so forth). Photochemical methods have been reported to form crosslinks in collagen type I molecules. See, Barbara P. Chan et al., Journal of Surgical Research 108, 77-84 (2002).

In some embodiments, at least one energy absorber is used within the solder material to enhance heating efficiency and/or heat distribution within the solder material. Preferred energy absorbers for use in the present disclosure include near-infrared absorbing materials (e.g., materials displaying a peak absorbance within a NIR window) and RF absorbing materials (e.g., materials displaying a peak absorbance at radiofrequencies).

Examples of near-infrared absorbing materials that can be used in conjunction with the present disclosure include one or more of the following, among others: inorganic NIR-absorbing particles including NIR-absorbing carbon particles such as carbon black, graphite, carbon fullerenes, carbon nanocubes and carbon buckeyballs, NIR-absorbing metal particles such as metal nanoshells, including noble metal nanoshells, such as gold nanoshells (e.g., gold-shell silica core nanoshells, gold-gold sulfide nanoshells, hollow gold nanoshells, and so forth), metal nanorods and nanowires including noble-metal nanorods and nanowires such as gold nanorods and nanowires, noble metal nanocages including gold nanocages, other NIR-absorbing noble metal nanoparticles, NIR-absorbing metal oxide particles, NIR-absorbing quanum dots, NIR-absorbing polymers including conductive polymers, India ink, NIR-absorbing chromophores including cyanine-based dyes (e.g., indocyanine green), polymethine-based dyes, azulenium-based dyes, squalirium-based dyes, thiopyrylium-based dyes, naphthoquinone-based dyes, anthraquinone-based dyes, various other polyaromatic dyes, phthalocyanine compounds, naphthalocyanine compounds, extended porphyrins, conjugated polyenes, polymethines and other chromophores.

Near-infrared absorbing materials have been used *in vivo* in tissue ablation procedures. See, e.g., D. Patrick O'Neal et al., "Photo-thermal tumor ablation in mice using near infrared-absorbing nanoparticles", Cancer Letters, Volume 209, Issue 2, 25 June 2004, Pages 171-176 describes use of near infrared (NIR) absorption of gold nanoshells, a class of gold nanoparticles with tunable optical absorptivities, for thermal ablation of tumors. See also Mary K. Popp, et al., "Photothermal Therapy Using Gold Nanorods and Near-Infrared Light in a Murine Melanoma Model Increases Survival and Decreases Tumor Volume," Journal of Nanomaterials, Volume 2014 (2014), Article ID 450670, 8 pages, which show that a NIR light-emitting diode (LED) light source can effectively and quickly heat gold nanorods to produce ablative temperatures *in vivo.* See further Yuanyuan Su et al., "Gold Nanoparticles-Decorated Silicon Nanowires as Highly Efficient Near-Infrared Hyperthermia Agents for Cancer Cells Destruction," Nano Lett., 2012, 12 (4), pp 1845-1850. Near-infrared absorbing dye (indocyanine green) has been used to enhance tissue welding in a system employing a solid albumin sheet and 800-nm wavelength diode laser. H. Xie, et al., "Thermal damage control of dye-assisted laser tissue welding: effect of dye concentration," in SPIE Proceedings of Lasers in Surgery: Advanced Characterization, Therapeutics and Systems XI, R. R. Anderson et al., 4244, pp. 189-192 (2001).

Examples of RF absorbing materials that can be used in conjunction with the present disclosure include one or more of the following, among others: particles of electrically conductive material, such as gold, copper, magnesium, iron, or other metal, including metal tubules, such as silver or gold nanotubes or silver or gold microtubes, magnetic particles including ferromagnetic materials such as iron, nickel, cobalt, iron alloys, nickel alloys, cobalt alloys, and steel, and ferrimagnetic materials such as magnetite, nickel-zinc ferrite, manganese-zinc ferrite, and copper-zinc ferrite, SPIONs (superparamagnetic iron oxide nanoparticles), particles made of piezoelectric crystal (natural or synthetic), carbon particles such as fullerenes, carbon nanotubes, other molecules or compounds having one or more graphene layers, and other RF-absorbing carbon molecules and compounds e.g., C60 (also known as a "buckyball" or a "buckminsterfullerene"), C70, C76, C84, buckytubes (single-walled carbon nanotubes, SWNTs), multi-walled carbon nanotubes (MWNTs), and other nano-sized or micro-sized carbon cage molecules and compounds, semiconductor nanoparticles (e.g., quantum dots), very small LC circuits (e.g., parallel LC tank circuits) other very small tuned (oscillatory) circuits, hollow particles (e.g., liposomes, magnetic liposomes, glass beads, latex beads, other vesicles made from applied materials, microparticles, microspheres, etc.) containing other substances (e.g., small particles containing argon or some other inert gas or other substance that has a relatively high absorption of electromagnetic energy).

As with near-infrared absorbing materials, RF absorbing materials have been used for *in vivo* ablation procedures. See, e.g., Evan S Glazer et al., "Non-invasive radiofrequency ablation of malignancies mediated by quantum dots, gold nanoparticles and carbon nanotubes," Ther Deliv. 2011 October ; 2(10): 1325-1330; Christopher J Gannon, et al., "Intracellular gold nanoparticles enhance non-invasive radiofrequency thermal destruction of human gastrointestinal cancer cells," Journal of Nanobiotechnology 2008 Jan 30, 6:2: Yang Xu et al. "Cobalt nanoparticles coated with graphitic shells as localized radio frequency absorbers for cancer therapy," Nanotechnology, 2008 Oct 29;19(43):435102.

In various embodiments, the energy absorbing materials are nanoparticles (e.g., nanospheres, hollow and filled nano shells, nanorods, hollow and filled nanotubes, etc.). A nanoparticle is defined herein as a particle having at least one dimension (i.e., length, width, height, diameter) in the range of 1 nm to 1000 nm, for example ranging from 1nm to 2 nm to 5 nm to 10 nm to 25 nm to 50 nm to 100 nm to 250 nm to 500 nm to 1000 nm (i.e., ranging between any two of the preceding values). Nanoparticle shape and dimensions may be optimized for peak absorption at a RF or NIR wavelength of choice. The energy absorbing materials are conductive nanoparticles such as metal or carbon nanoparticles in various embodiments.

In various aspects of the present disclosure, NIR-absorbing materials are used to bond mesh materials to tissue surfaces, for example, by the application of NIR light of a suitable wavelength to a NIR-absorbing material and a solder material (e.g., a biological solder material, including those set forth above, among others) in intimate association with a mesh material and a tissue surface (e.g., a NIR-absorbing material admixed with a solder material or coated on a surface of a solder material that is in contact with and disposed between mesh material and tissue), such that the mesh material is fastened onto one or more desired locations on the tissue surface.

In various aspects of the present disclosure, RF-absorbing materials are used to bond mesh materials to tissue surfaces, for example, by the application of RF energy of a suitable wavelength to a RF-absorbing material and a solder material (e.g., a biological solder material, including those set forth above, among others) in intimate association with a mesh material and a tissue surface (e.g., a RF-absorbing material admixed with a solder material or coated on a surface of a solder material that is in contact with and disposed between mesh material and tissue), such that the mesh material is fastened onto one or more desired locations on the tissue surface.

An advantage of using RF or NIR irradiation or is that there is less risk of causing damage to the tissue (cell death) from heat, due to the relative transparency of tissue energy at these wavelengths. Another advantage of using RF or NIR irradiation to achieve mesh-to-tissue bonding is that complications due to uneven heat distribution may be reduced or eliminated.

Mesh materials for use in accordance with the present disclosure include various synthetic and natural polymers. Beneficial polymers for forming mesh fiber(s) may be selected from the following, among others: (a) polyolefin homopolymers and copolymers, including homopolymers and copolymers of C2-C8 alkenes, for example, polyethylene and polypropylene among others, (b) fluoropolymers, including homopolymers and copolymers of C2-C8 alkenes in which one or more hydrogen atoms are substituted with fluorine, for example, polytetrafluoroethylene (PTFE), polyvinylidene fluoride (PVDF), poly(vinylidene fluoride-co-hexafluoropropene) (PVDF-HFP) among others, (c) polyamides such as nylons, among others, (d) polyesters, including, for example, polyethylene terephthalate, among others, (e) polyurethanes such as polyisobutylene based polyurethanes (PIB-PU) that comprise one or more polyisobutylene segments, among others, (f) polyoxyalkylenes including homopolymers of trioxane (e.g., polytrioxane, also known as polyoxymethylene or acetal) and copolymers of trioxane (e.g., copolymers of trioxane and dioxane) and (g) styrenic copolymers such as alkene-styrene copolymers, including block copolymers comprising one or more polystyrene blocks and one or more polyalkene blocks, for instance, poly(styrene-*b*-isobutylene-*b*-styrene) (SIBS), poly(styrene-*b*-ethylene/butylene-*b*-styrene) (SEBS).

Mesh materials are commonly in the form of fibers. Consequently, surgical meshes in accordance with the present disclosure may be formed using a variety of fiber-based construction techniques and include, for example, woven meshes and non-woven meshes (e.g., knitted, braided, coiled, randomly wrapped, spunbound, etc.).

The overall fiber width (e.g., the diameter of circular fibers) in the meshes of the present disclosure may vary widely. In certain embodiments, the surgical meshes of the present disclosure may have fiber widths ranging from 50 µm to 200 µm, for example, from 50 µm to 70 µm to 90 µm to 110 µm to 130 µm to 150 µm to 170 µm to 190 µm to 200 µm.

In certain embodiments, fibers may be provided with surface features, for example, to increase the surface area of the fibers and thus the contact area between the fibers and a solder material coating.

Surgical meshes in accordance with the present disclosure may also have a wide range of pore sizes. In various embodiments, the surgical meshes of the present disclosure may have area pore sizes ≥ 0.25mm². In various embodiments, the pore size can be varied, for example, to modify tissue in-growth properties. Meshes include Type I meshes, which are macroporous prostheses (i.e., with pores >75µm), Type II meshes, which are microporous prostheses (i.e., pores <10µ) and Type III meshes, which are macroporous prostheses with multifilamentous or microporous components.

In certain embodiments, meshes may be formed with differing pore sizes in differing areas of the mesh. This may lead to differing tissue ingrowth characteristics in some parts of the mesh relative to others, and may lead to different mesh-tissue interactions (e.g. based on the flexibility of the mesh and tissue). This may also result in differing bonding material densities in some parts of the mesh relative to others. For example, areas of smaller pore size are associated with a higher fiber density and thus higher fiber surface area, which can allow for increased bonding material to be coated onto the fibers in those areas, relative to areas of larger pore size. Higher bonding material density may, in turn, be associated with enhanced bonding in those areas, relative to areas of larger pore size.

In certain embodiments, meshes may be provided with one or more loops along the edges of the mesh (e.g., at the end(s) of one or more arms) to facilitate unraveling and placement of the mesh once inside the body. The loops may be made, for example, of a suitable degradable material.

Bonding material may be associated with mesh material in various ways. For example, mesh material may be admixed or impregnated with bonding material, mesh material may be coated with bonding material, a sheet of bonding material may be laminated to mesh material, and so forth.

The bonding material may be present throughout the entire mesh or on only a portion of the mesh, for instance, associated with 1 % or less to 99% or more of the total length of fiber within the mesh (e.g., from 1% to 5% to 10% to 25% to 50% to 75% to 90% to 95% to 99%) (i.e., ranging between any two of the preceding values). The bonding material may be present, for example, on only one side of the mesh but not the other, or may be present only in certain lateral portions of the mesh but not others, for example, being present only at the ends of the mesh, present only at strategic zones along the edges of the mesh, and so forth. In certain embodiments, this allows the mesh to be largely free of bonding material, which reduces bonding material consumption and potentially facilitates tissue growth around the mesh in areas free of bonding material, while nonetheless ensuring that the mesh can be securely attached in those areas where bonding material is present.

In certain embodiments, differing bonding materials may be employed in different areas of the mesh. As one example, different areas of the mesh may be coated with bonding materials having different compositions (e.g. bonding materials having different bioabsorption rates, etc.).

Mesh material may be impregnated and/or coated with bonding material either before a mesh is formed (e.g., when the mesh material is in fiber form) or after a mesh is formed. Mesh material may be impregnated and/or coated with bonding material, for example, using various techniques which may be selected, for example, from dipping techniques, spraying techniques, spin coating techniques, web coating techniques, electrostatic techniques, techniques in which bonding material is selectively applied to certain regions of the mesh but not others, for example, through the use of a suitable application device such as a sprayer, brush, roller, pen, or printer (e.g., screen printing device, ink jet printer, etc.). A partial or complete bonding material coating may also be formed by coextruding the mesh material with bonding material.

Meshes may be provided with an intermediate layer of material between a layer of bonding material and the mesh material, for instance, an intermediate layer may be formed of a suitable polymer, such as an isobutylene-styrene copolymers (e.g., SIBS), among many others.

In certain embodiments, meshes in accordance with the present disclosure may be at least partially covered with a removable thin film. This film prevents the mesh from sticking to itself or to other objects during delivery. The film may be peeled off the mesh once in use, for example, inside the body. The film may comprise, for example, a material such as a fluoropolymer (see, e.g., the fluoropolymers listed above). The film may be provided with features that allow the film to be more easily gripped (e.g., loops, etc.), thereby facilitating removal of the film from the mesh. In other embodiments, the film may be formed from a soluble material that can be removed upon application of a suitable fluid, for example, saline, among other possibilities. In still other embodiments, the film may be formed from a material that degrades upon application of energy that is employed to activate the bonding material.

Surgical meshes in accordance with the present disclosure include a variety of meshes for soft tissue repair, including meshes for pelvic floor repair, vaginal slings, meshes for renal pelvis repair, urethral slings, meshes for fecal incontinence, hernia meshes (e.g., meshes for inguinal hernia, hiatus hernia, etc.), meshes for thoracic wall defects, breast support meshes and various other soft-tissue surgical support meshes, including meshes for cosmetic and reconstructive surgery, among others. Depending on the procedure, meshes in accordance with the present disclosure may be implanted by one or more of the following routes, among others: transluminally, for instance, transvaginally (e.g., by vaginal incision), laparoscopically, via open abdominal surgery (laparotomy), transrectally, transcystoscopically, and percutaneously. Surgical meshes may be surgically implanted in a variety of subjects, typically vertebrate subjects, more typically mammalian subjects, including human subjects, pets and livestock.

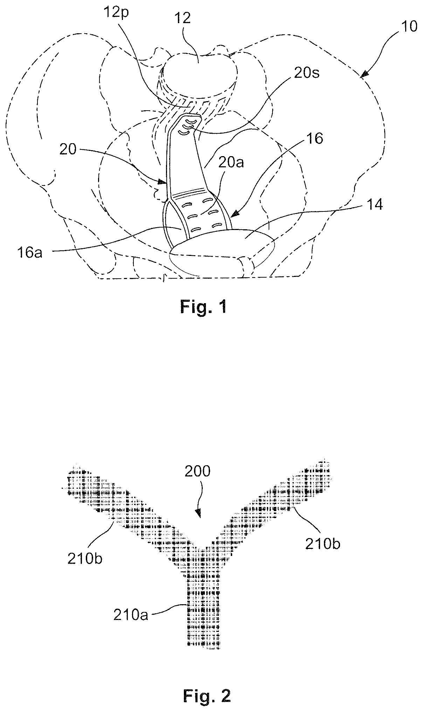

Turning now to Fig. 2, there is schematically illustrated therein a surgical mesh 200, specifically a Y-shaped mesh, having arms 210a and 210b. The mesh 200 may comprise a fiber mesh material which is associated with a bonding material (e.g., a solder material associated with an energy absorbing material, etc.), for instance, a coating of bonding material that covers all or a portion of the mesh, among other possibilities.

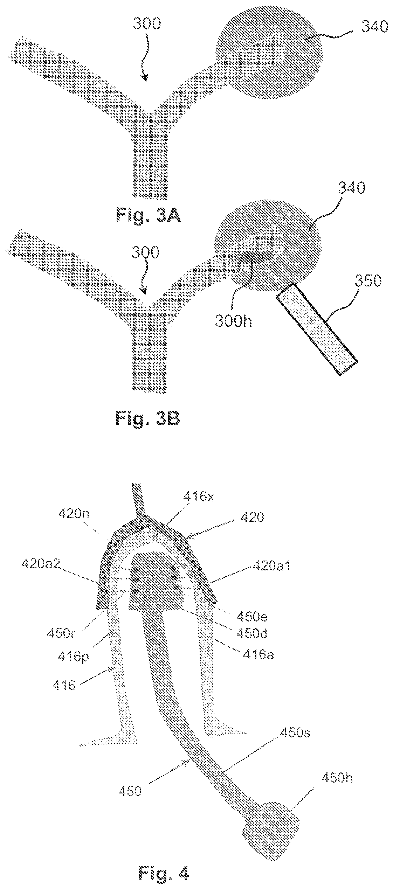

Various methods may be used in accordance with the present disclosure to attach meshes to tissue. As previously noted, these methods reduce or avoid the need for mechanical fasteners such as sutures, staples, and so forth. Figs. 3A-3B are schematic illustrations of a medical procedure for attaching a surgical mesh, in accordance with an embodiment of the present disclosure. As shown in Fig. 3A, a mesh 300 with an associated bonding material, such as a solder material and/or energy absorbing material, is placed in contact with tissue 340. Then, as shown in Fig. 3B, an energy source 350 is used to supply energy (e.g., NIR radiation or RF radiation) to the mesh 300 and tissue 340 in area 300h, causing the mesh 300 to become attached to the tissue 340 in area 300h. The entire mesh or only a portion thereof may be associated with bonding material as described elsewhere herein. As previously noted, the use of NIR radiation or RF radiation of suitable wavelengths minimizes energy absorption by the tissue, reducing the likelihood of tissue damage (i.e., burning) due to energy absorption.

In the preceding embodiment, the mesh and the bonding material are applied to the tissue simultaneously (e.g., the mesh material is coated, impregnated or otherwise associated with the bonding material). In some embodiments, bonding material is applied to the site independently of the mesh, in which case the mesh either may be associated with bonding material at the time of application or free of bonding material at the time of application. In these embodiments, the bonding material may be applied to tissue followed by the mesh, or the mesh may be applied to tissue followed by the bonding material. The independently applied bonding material may be applied in solid form, liquid form or a combination thereof. For instance, the independently applied bonding material may be applied in the form of a fluid such as a liquid, paste or gel (e.g., an organic or aqueous liquid, paste or gel comprising a solider material and/or energy absorbing material) or may be applied as a solid (e.g., a porous or non-porous film that includes a solder material and/or energy absorbing material, for instance, a solid film with raised sections (e.g., similar to a LEGO block) that can be fitted/plugged into the mesh).

As noted above, meshes are attached to tissue during the course of a variety of surgical procedures, including various urogynecological procedures. In this regard, pelvic floor disorders are commonly treated by implanting a surgical mesh within the patient's pelvis to support the organ or organs that require support. For example, vaginal prolapse is a common condition causing symptoms such as a sensation of dragging or fullness in the vagina, difficulty emptying the bowel or bladder, and back ache. About 1 in 10 women need surgery for prolapse of the uterus or vagina. Sacrocolpopexy is one procedure that may be used to repair pelvic organ prolapse. This procedure can be performed, for example, using an open abdominal technique or through the use of minimally invasive surgery, such as laparoscopy, trans-vaginal surgery, or combinations of the same.

More specifically, sacrocolpopexy is a procedure to correct prolapse of the vaginal vault (top of the vagina) in women who have had a previous hysterectomy (uterus removal). If the cervix is also removed during your surgery, the top of the vagina that is sewn together is called the "vaginal cuff." Turning to Fig. 1, there is shown a female human pelvis 10 including the sacrum 12, bladder 14 and vagina 16. During a sacrocolpopexy procedure, the vagina 16 is typically freed from the bladder 15 along the anterior surface 16a and from the rectum along the posterior surface (not shown). Then a surgical mesh 12, typically a y-shaped mesh having three arms is implanted. In particular, one arm 20s is attached to the sacral promontory 12p, another arm 20a is attached to the anterior surface 16a of the vagina 16 and a third arm (not shown) is attached to an posterior surface (not shown) of the vagina 16, thereby suspending the apex of the vagina 16 to the sacral promontory 12p in a manner that mimics the anatomic support naturally provided by the uterosacral and cardinal ligaments. Thus, where a Y-shaped mesh such as that shown in Fig 2 is employed, one arm of the mesh (e.g., one of arms 210b) is attached to the anterior vaginal wall, another arm (e.g., the other of arms 210b) is attached to the posterior vaginal wall, and a third arm (e.g., arm 210a) is attached to tissues adjacent the sacrum, for example, the sacral promontory.

In the present disclosure, the arms are attached using tissue bonding, which may save significant amounts of time and improve implanted mesh properties relative to current methods in which the mesh is attached to tissue using sutures. For example, in a typical procedure, approximately 6 suture knots are used to attach a mesh portion to each side of the vaginal wall. The creation of suture knots is time-consuming, typically consuming between 3-8 minutes per knot. Moreover, in procedures where sutures are used to attach meshes, a mesh may bunch, which can constrain the mesh properties.

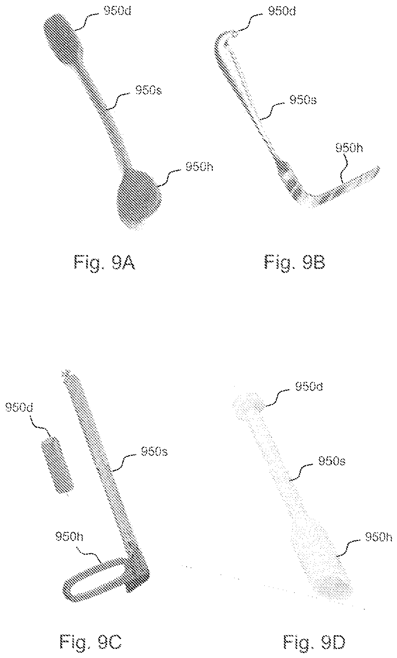

Devices suitable for vaginal manipulation during sacrocolpopexy are presently known and include, for example, Colpassist™ vaginal positioning device available from Boston Scientific (see Fig. 9A), a Heaney Retractor handle (see Fig. 9B), a Cooper Surgical's Sacrocolpopexy Tip with Rumi handle (Fig. 9C) or Colpo-Probe™ Vaginal Fornix Delineator (Fig. 9D). Each of these comprises a handle 950h, an elongate shaft 950s, and a distal portion 450d for supporting the vaginal apex during the surgical procedure. A vaginal manipulator device is used to impart a stable structure to the vagina during the procedure, improving the ease with the mesh can be placed onto the anterior and/or posterior vaginal walls. In some aspects of the present disclosure, the difficulty associated with mesh suturing is reduced by pairing an energy source useful in the soldering procedures with a vaginal manipulator device. After the mesh and bonding material are suitably positioned, the vaginal manipulator device is used to irradiate the mesh and bonding material through the vaginal wall.

Fig. 4 is a schematic illustration showing a vaginal manipulator device 450 in accordance with the present disclosure positioned inside the vagina 416. The vaginal manipulator 450 includes a handle 450h, an elongate shaft 450s, and an energy emitting distal portion 450d which includes a plurality of energy emitting elements 450e (e.g., a plurality of NIR LED's, NIR lasers, NIR laser diodes, NIR transmitting optical fiber etc.). The energy emitting elements 450e are configured to emit radiation 450r to which the vaginal tissue is substantially transparent such that a significant amount of the radiation 450r passes through the posterior vaginal wall 416p and anterior vaginal wall 416a. Thus, the vaginal manipulator 450 may be designed to target the bonding material and pass through tissue without damaging the tissue by operating within a wavelength range that minimally affects tissue. Energy may be irradiated from the sides and/or distal end of the vaginal manipulator device 415, thereby allowing the vaginal manipulator device 415 to irradiate both the anterior and posterior vaginal walls at the same time. A Y-shape mesh 420 is positioned at the vaginal apex 416x. One arm 420a1 is positioned on the anterior vaginal wall 416a whereas another arm 420a2 is positioned on the posterior vaginal wall 416p. The mesh 416 is provided with a bonding material that includes a solder material and an energy absorbing material (e.g., nanoparticles 420n) that is tuned to absorb the radiation 450r emitted from the energy emitting elements 450e of the vaginal manipulator 450.

Such a configuration is desirable in a laparoscopic context in that, by combining the energy source and vaginal manipulator, there are fewer devices to be introduced through laparoscopic port. Moreover, by allowing the vaginal manipulator to irradiate the mesh through tissue to which it is already adjacent, such a configuration should further shorten procedure time by reducing the time spent maneuvering the energy source. In this regard, irradiating both the anterior and posterior vaginal walls can be a challenge, requiring the user to shift placement of the energy source around the vagina and, given that there is a limited amount of space inside a laparoscopic port, maneuvering and maintaining constant distance becomes difficult.

Thus, in one aspect, the present disclosure provides the ability to use a bonding material containing a tissue solder and an energy absorbing material, such as a NIR or IR absorbing material (e.g., suitably tuned nanoparticles), in conjunction with a vaginal manipulator, which can be used in open or laparoscopic procedures to position the vagina and irradiate bonding material uniformly through tissue. The vaginal manipulator device is inserted into the vagina and is positioned to a desired location. Once the bonding material and mesh are placed on the opposite side, the manipulator is activated such that it irradiates the solder through the tissue. After the desired amount of energy has been absorbed by the bonding material, the vaginal manipulator ceases irradiating energy and is removed from the vagina. In comparison with procedures in which separate devices are employed (i.e., a vaginal manipulator positioned in the vagina and an energy source positioned on the other side of the vaginal wall to irradiate the solder), by associating the energy source with the vaginal manipulator, the present disclosure reduces the number of devices required, simplifies the procedures, and reduces the steps used to tissue solder the mesh to the vaginal wall.

In certain embodiments, the present disclosure includes a method having the following steps: (A) An energy-emitting vaginal manipulator device is placed within the vagina and is positioned by the user. (B) A mesh and bonding material (e.g., a mesh coated with bonding material containing an energy absorbing material and a solder material) is placed onto the vaginal tissue wall on the opposite side from the vaginal manipulator device. (C) The user activates the vaginal manipulator device and energy is emitted from one or more energy sources located at the distal end of the vaginal manipulator device though the vaginal tissue. (D) The energy absorbing material (e.g., nanoparticles, dye, etc.) in the bonding material absorbs the energy and generates heat, which activates solder material in the bonding material, resulting in a bond (e.g., a crosslinked bond) between the mesh and the vaginal wall tissue. (E) After the desired amount of energy has been absorbed by the bonding material, the energy source in the vaginal manipulator device is shut down, and the user then removes the vaginal manipulator device from the vagina.

In some embodiments, the vaginal manipulator device is a stand-alone device, which may be powered, for example, by rechargeable or disposable batteries, or by an external source of AC or DC power.

In some embodiments, the vaginal manipulator device may be connected to an external control unit, which controls the energy emitting from the energy source. The control unit may include a suitable source of AC or DC power for activating energy generating components in the vaginal manipulator device, for example, NIR generating components such as NIR LEDs, NIR laser diodes, NIR lasers, an RF generator, and so forth. The control unit may contain a NIR generator in which case NIR energy may be conducted to the vaginal manipulator device via one or more NIR-transmitting optical fibers. The control unit may include a radiofrequency energy generator which directs RF energy to RF energy irradiators associated with the vaginal manipulator device. In some embodiments, the control unit is designed to accept user input (e.g., via physical buttons, touchscreen, etc.), thereby allowing treatment parameters to be set by a health care provider.

In some embodiments, the energy source is controlled without the use of a sensor. For instance, the energy source may be controlled based on the experience of the user. For example, the energy source may be activated by a user via a control on the vaginal manipulator device, via a control on the control unit, or via a control foot pedal, among other possibilities.

The energy source may be controlled based on a suitable energy output algorithm. In some instances, the vaginal manipulator device is preset to produce a certain energy output profile that will irradiate the solder but not damage the tissue, in which case the device may or may not allow the user to change settings. In some instances, an automatic timer (e.g., in the vaginal manipulator device or control unit) may be provided, which shuts off the energy source after a set amount of time has expired, thereby preventing user error in which the energy source is activated for a time period that is too long and leads to unintentional tissue ablation. The vaginal manipulator device or control unit may be configured to generate an audible sound while the energy source is activated to alert the user to activation of the device. The vaginal manipulator device or control unit may be configured to generate a visible signal (e.g., via a visible light source such as an LED) while the energy source is activated to alert the user to activation of the device. For example, light source may be located in the handle of the vaginal manipulator device, which is illuminated to show that the device is active.

In other embodiments, a sensor is used in conjunction with the energy source to provide feedback regarding the amount of energy being directed to the bonding site, and this feedback can be used to adjust the energy source output. For example, in certain embodiments, the sensor is a temperature sensor which detects the amount of heat at the bonding site. In these embodiments, suitable software can be employed to adjust the output of the energy source based on input from the thermal sensor. The sensor may be provided, for example, in the same device as the energy source. For example, the thermal sensor may be integrated into the vaginal manipulator device, measuring the temperature at an internal surface of the vaginal wall, thereby ensuring that sufficient energy is being provided for bonding, while also preventing significant thermal ablation. The thermal sensor may also be provided in a separate device. For instance, a thermal sensor may be used to measure the temperature on the opposite side of the vaginal wall to supply data relating to the solder temperature (e.g., sending data back to the control unit). In this regard, a thermal sensor may be provided, for example, in a medical device that is used for mesh delivery or in a separate device that introduced via a suitable route (e.g., laparoscopically). The thermal sensor may be, for example, in the form of a separate handheld unit that is used to monitor the temperature of the bonding material. The thermal sensor may be, for example, in the form of device that can be wrapped around the vaginal wall on top of the mesh and bonding material. The device may contain multiple sensors (e.g., multiple thermocouples) for this purpose.

As previously indicated, a plurality of energy sources may be provided at a distal end of the vaginal manipulator device, allowing a wider area to be irradiated. In some embodiments, a user is able to control of the area of irradiation by selectively turning on or off the energy sources. In some embodiments, a control mechanism in the vaginal manipulator device or control unit is used to selectively turning on or off the energy sources, for example, based sensor data. The energy source(s) may be located at the tip and/or the sides of the distal end of the vaginal manipulator.

The energy source(s) may be recessed into the vaginal manipulator body, which may be useful where surface(s) of the energy source(s) is/are hot enough to burn tissue upon direct contact with tissue. The energy source(s) may be provided on a separate inner shaft that is configured to be placed inside a main body of the vaginal manipulator device, which acts like a sheath for the energy source(s). This allows the energy source(s) to be moved longitudinally (e.g., up and down) and rotated to irradiate various areas of the vagina. For example, the inner shaft may be moved longitudinally and rotated by means of a protrusion (i.e., a handle) that extends out of the vaginal manipulator device near the bottom of the vaginal manipulator device. Movement of the inner shaft may be also controlled by a lever or trigger that moves the inner shaft longitudinally, depending on the positioning of the lever or trigger.

The vaginal manipulator device may contains a lumen for suction, which may be used to pull tissue towards the device, reducing the distance the energy source has to travel. In these embodiments, the vaginal manipulator device may be provided with a balloon proximal to the suction lumen to close off the vaginal opening and help generate suction.

The distal end of the vaginal manipulator device may be provided with thin spikes that create small punctures through the vaginal wall in some embodiments. By providing energy sources (e.g., NIR sources, RF sources, etc.) on these spikes, the device is able to provide irradiation on the same side as the bonding material. Holes generated by spikes may be small enough to heal quickly. The device may be provided with a mechanism to deposit a closure material on the inside of the vaginal wall to help close up small punctures that are created. Examples of spikes include (a) spikes that curve toward the bonding material so that the energy source may target the bonding material directly, (b) spikes that emit radiation from the sides, (c) spikes that contain front-firing energy sources, whereas the interior of the tips of the spikes contain a reflective surfaces that reflect energy from the energy sources onto tissue, (d) spikes that can be rotated to bond a greater area, in which case all spikes may articulate uniformly with a single action or may be independently articulated, and (e) spikes that are recessed within the vaginal manipulator device body during insertion and that are deployed outwardly after insertion, for example, based on user action that deploys spikes with enough force to puncture the vaginal wall. The vaginal manipulator and spikes may be put in place (punctured through the vaginal wall) before the mesh is placed, allowing the spikes to assist in mesh placement.

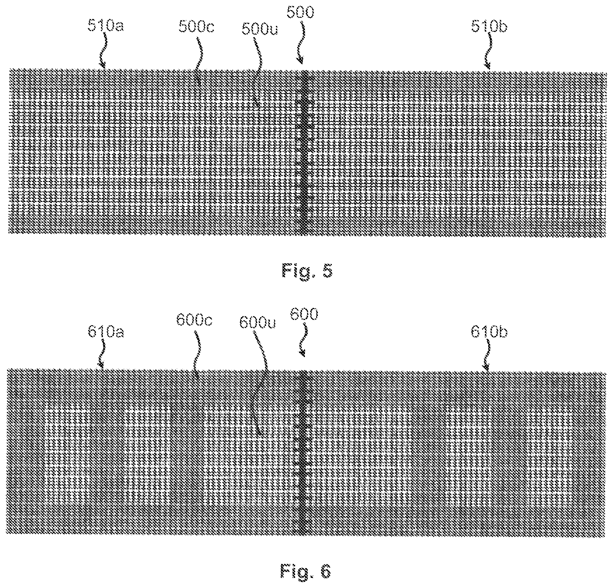

As previously noted, bonding material may be associated with an entire mesh or with only a portion of a mesh. For instance, in some embodiments, bonding material may be applied to only one side of a mesh. In other embodiments, bonding material may be associated with certain portions along the length of the mesh, but not others. For example, in accordance with an embodiment illustrated in Fig. 5, a mesh 500 is shown having arms 510a, 510b. In Fig. 5, certain portions 500c of the mesh 500 are provided with bonding material (i.e., portions along the lateral edges of the arms) whereas the remaining portions 500u of the mesh 500 are not provided with bonding material. In one particular embodiment, the mesh is a Y-shaped mesh wherein the arms 510b (one arm shown) are provided with bonding material and are configured, for example, for attachment to the vaginal anterior and/or posterior wall, while the arm 510a is provided with bonding material and is configured, for example, for attachment to tissues adjacent the sacrum, for example, the sacral promontory, in a sacrocolpopexy procedure. In some embodiments, the arms 510b (one arm shown) are provided with bonding material for attachment to the vaginal anterior and/or posterior wall of the vagina, whereas the arm 510a, on the other hand, is configured for suturing to tissues adjacent the sacrum, in which case arm 510a need not be provided with bonding material.

In accordance with another embodiment illustrated in Fig. 6, a mesh 600 is shown having arms 610a, 610b. In Fig. 6, certain portions 600c of the mesh 600 are provided with bonding material (i.e., portions along the lateral edges of the arms, portions along the end edges of the arms, and portions traversing the arms) whereas remaining portions 600u of the mesh 600 (e.g., certain interior areas of the mesh) are not provided with bonding material. As with the mesh of Fig. 6, in one particular embodiment, the mesh is a Y-shaped mesh wherein the arms 610b (one arm shown) are provided with bonding material and are configured, for example, for attachment to the vaginal anterior and/or posterior wall, while the arm 610a is provided with bonding material and is configured, for example, for attachment to tissues adjacent the sacrum, for example, the sacral promontory, in a sacrocolpopexy procedure. In some embodiments, the arms 610b (one arm shown) are provided with bonding material for attachment to the vaginal anterior and/or posterior wall of the vagina, whereas the arm 610a, on the other hand, is configured for suturing to tissues adjacent the sacrum, in which case arm 610a need not be provided with bonding material.

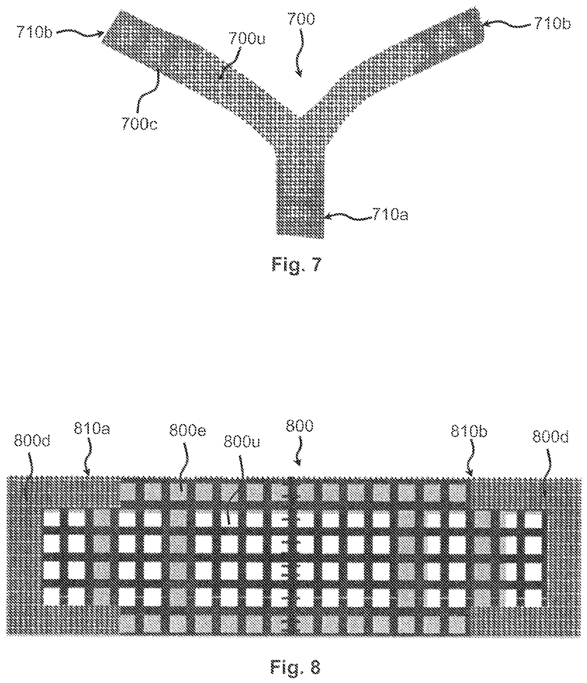

Another mesh of this type is illustrated in Fig. 7, in which a Y-shaped mesh 700 is shown having arms 710a, 710b in which some portions 700c of the mesh 700 are provided with bonding material (i.e., portions along the lateral edges near the ends of the arms, portions along the end edges of the arms, and portions traversing the arms) while other portions 700u of the mesh 700 are not provided with bonding material. As above, in a particular embodiment, the mesh is a Y-shaped mesh wherein the arms 710b (one arm shown) are provided with bonding material and are configured, for example, for attachment to the vaginal anterior and/or posterior wall, while the arm 710a is provided with bonding material and is configured, for example, for attachment to tissues adjacent the sacrum, for example, the sacral promontory, in a sacrocolpopexy procedure. In some embodiments, the arms 710b (one arm shown) are provided with bonding material for attachment to the vaginal anterior and/or posterior wall of the vagina, whereas the arm 710a, on the other hand, is configured for suturing to tissues adjacent the sacrum, in which case arm 710a need not be provided with bonding material.

In accordance with yet another embodiment illustrated in Fig. 8, a mesh 800 is shown having arms 810a, 810b. In Fig. 8, some portions 800e of the mesh 800 are of larger pore size and are provided with bonding material (i.e., portions along lateral edges of the arms and portions traversing the arms), other portions 800d of the mesh 800 are of smaller pore size and are provided with bonding material (i.e., portions along lateral edges of the arms and portions along end edges of the arms), and still other portions 800u of the mesh 800 (e.g., portions in the interior of the mesh) are not provided with bonding material. As with the meshes in Figs. 5-7, in a particular embodiment, the mesh is a Y-shaped mesh wherein the arms 810b (one arm shown) are provided with bonding material and are configured, for example, for attachment to the vaginal anterior and/or posterior wall, while the arm 810a is provided with bonding material and is configured, for example, for attachment to tissues adjacent the sacrum, for example, the sacral promontory, in a sacrocolpopexy procedure. In some embodiments, the arms 810b (one arm shown) are provided with bonding material for attachment to the vaginal anterior and/or posterior wall of the vagina, whereas the arm 810a, on the other hand, is configured for suturing to tissues adjacent the sacrum, in which case arm 810a need not be provided with bonding material.

In certain embodiments, the meshes of the present disclosure may comprise various additional agents other than those discussed above, including therapeutic agents and imaging agents, among other possible agents. Such agents may be, for example, incorporated into all or a portion of the mesh material, or such agents may be applied in a coating (e.g., admixed with a bonding material or applied independently of a bonding material) over all or a portion of the mesh material, among other strategies.

"Therapeutic agents," drugs," "bioactive agents" "pharmaceuticals," "pharmaceutically active agents" and other related terms may be used interchangeably herein. Therapeutic agents may be used singly or in combination.

In certain embodiments, the meshes of the present disclosure may comprise one or more therapeutic agents, for example, selected from the following, among many others: (a) female hormones such as estrogen (including estrogen cocktails) and progesterone, (b) anti-inflammatory agents including corticosteroids such as hydrocortisone and prednisolone, and non-steroidal anti-inflammatory drugs (NSAIDS) such as aspirin, ibuprofen, and naproxen; (c) narcotic and non-narcotic analgesics and local anesthetic agents (e.g., for purposes of minimizing pain); (d) growth factors such as epidermal growth factor and transforming growth factor-α (e.g., for purposes of stimulate the healing process and or promoting growth of collagenous tissue); (e) antimicrobial agents including chlorhexidine, triclosan, nitrofurazone, benzalkonium chlorides, silver salts, silver particles, metallic silver and antibiotic agents such as the penicillins (e.g., penicillin G, methicillin, oxacillin, ampicillin, amoxicillin, ticarcillin, etc.), the cephalosporins (e.g., cephalothin, cefazolin, cefoxitin, cefotaxime, cefaclor, cefoperazone, cefixime, ceftriaxone, cefuroxime, etc.), the carbapenems (e.g., imipenem, metropenem, etc.), the monobactems (e.g., aztreonem, etc.), the carbacephems (e.g., loracarbef, etc.), the glycopeptides (e.g., vancomycin, teichoplanin, etc.), bacitracin, polymyxins, colistins, fluoroquinolones (e.g., norfloxacin, lomefloxacin, fleroxacin, ciprofloxacin, enoxacin, trovafloxacin, gatifloxacin, etc.), sulfonamides (e.g., sulfamethoxazole, sulfanilamide, etc.), diaminopyrimidines (e.g., trimethoprim, etc.), rifampin, aminoglycosides (e.g., streptomycin, neomycin, netilmicin, tobramycin, gentamicin, amikacin, etc.), tetracyclines (e.g., tetracycline, doxycycline, demeclocycline, minocycline, etc.), spectinomycin, macrolides (e.g., erythromycin, azithromycin, clarithromycin, dirithromycin, troleandomycin, etc.), and oxazolidinones (e.g., linezolid, etc.), (f) anti-collagenase agents (collagenase inhibitors) including tetracycline compounds such as doxycycline, bacitracin, prednisolone, medroxyprogesterone, cysteine, acetylcysteine, N-acetylcysteine, sodium citrate, edetic acid (EDTA), TIMP-1 and TIMP-2, (g) anti-elastase agents (elastase inhibitors) such as alpha-1-protease inhibitor, alpha-1-antitrypsin, secretory leukocyte protease inhibitor, glycosaminoglycans (e.g., heparin), elastase inhibitor I, elastase inhibitor II, elastase inhibitor III, elastase inhibitor IV, peptide aldehydes, caffeic acid, elafin, ceramides, nicardipine, procyanidins, proanthocyanidins, coumarinic derivatives, sivelestat (e.g., sivelestat sodium salt), 6-amino-2-phenyl-4H-3,1-benzoxazin-4-one aminoacyl, and dipeptidyl derivatives, (h) pharmaceutically acceptable salts, esters and other derivatives of the foregoing, and (i) combinations of two or more of the foregoing.

Additional agents for use in conjunction with the meshes of the present disclosure also include imaging agents including (a) contrast agents for use in connection with x-ray fluoroscopy, including metals, metal salts and oxides (particularly bismuth salts and oxides), and iodinated compounds, among others, (b) contrast agents for use in conjunction with ultrasound imaging, including organic and inorganic echogenic particles (i.e., particles that result in an increase in the reflected ultrasonic energy) or organic and inorganic echolucent particles (i.e., particles that result in a decrease in the reflected ultrasonic energy), and (c) contrast agents for use in conjunction with magnetic resonance imaging (MRI), including contrast agents that contain elements with relatively large magnetic moment such as Gd(III), Mn(II), Fe(III) and compounds (including chelates) containing the same, such as gadolinium ion chelated with diethylenetriaminepentaacetic acid.

In various embodiments, the mesh may contain from less than 1 wt% to 50 wt% or more of one or more of the preceding additional agents.

In another aspect of the disclosure, medical kits useful in treating a patient in need of tissue repair are provided. The medical kits may include all or a subset of all the components useful for treating a patient. For example, the medical kits may comprise any combination of any two, three, four, or more of the following items: (a) a surgical mesh in accordance with the present disclosure, (b) a bonding material in accordance with the present disclosure, in liquid, paste or gel form (e.g., containing a solder material and a NIR- or RF-absorbing material dissolved and/or suspended in a solution, paste or gel) or in solid form (e.g., in the form of a continuous sheet, porous sheet, etc., that contains a solder material and a NIR- or RF-absorbing material), (c) a vaginal manipulator device (either with or without one or more associated energy sources), (d) a surgical instrument that can hold and place a surgical mesh, (d) an energy source (e.g., in a stand-along unit, associated with a mesh placement instrument, etc.), (e) suitable packaging material, and (f) printed material with one or more of the following: (i) storage information and (ii) instructions regarding how to implant the surgical mesh in a subject.

Although various embodiments are specifically illustrated and described herein, it will be appreciated that modifications and variations of the present disclosure are covered by the appended claims.

## Claims

1. A surgical mesh (300, 400, 500, 600, 700, 800) comprising a mesh material and a tissue bonding material associated with the mesh material that comprises a tissue solder and an absorber material, wherein the mesh is configured to bond to patient tissue when exposed to a near-infrared energy source while the surgical mesh is in contact with the tissue, wherein the absorber material comprises nanoparticles and is a near-infrared absorber material.

2. The surgical mesh of claim 1, wherein the near-infrared absorber material displays an absorbance peak between 650 nm and 950 nm.

3. The surgical mesh of claim 1, wherein the nanoparticles are selected from near-infrared absorbing metallic nanoparticles, near-infrared absorbing semiconductor nanoparticles, near-infrared absorbing carbon nanoparticles and near-infrared absorbing dyes.

4. The surgical mesh of any of claims 1-3, wherein the tissue solder is selected from chitosan, albumin, collagen, cellulose, elastin, nano-peptides, derivatives of the foregoing, and combinations of two or more of the foregoing.

## Patentansprüche

1. Chirurgisches Netz (300, 400, 500, 600, 700, 800), das ein Netzmaterial und ein mit dem Netzmaterial verbundenes Gewebebindungsmaterial aufweist, das ein Gewebelötmittel und ein Absorbermaterial umfasst, wobei das Netz zum Binden an Patientengewebe ausgebildet ist, wenn es einer Nah-Infrarot-Energiequelle ausgesetzt ist, während sich das chirurgische Netz mit dem Gewebe in Kontakt befindet, wobei das Absorbermaterial Nanopartikel aufweist und ein Nah-Infrarot-Absorbermaterial ist.

2. Chirurgisches Netz nach Anspruch 1, wobei das Nah-Infrarot-Absorbermaterial einen Absorptionspeak zwischen 650 nm und 950 nm zeigt.

3. Chirurgisches Netz nach Anspruch 1, wobei die Nanopartikel aus Nah-Infrarot-absorbierenden metallischen Nanopartikeln, Nah-Infrarot-absorbierenden Halbleiternanopartikeln, Nah-Infrarot-absorbierenden Kohlenstoff-Nanopartikeln und Nah-Infrarot-absorbierenden Farbstoffen ausgewählt sind.

4. Chirurgisches Netz nach einem der Ansprüche 1-3, wobei das Gewebelötmittel aus Chitosan, Albumin, Collagen, Cellulose, Elastin, Nanopeptiden, deren Derivaten und Kombinationen von zwei oder mehr davon ausgewählt ist.

## Revendications

1. Treillis chirurgical (300, 400, 500, 600, 700, 800) comprenant un matériau de treillis et un matériau liant les tissus associé avec le matériau de treillis qui comprend une soudure à tissus et un matériau absorbant, où le treillis est configuré pour se lier à un tissu d'un patient quand il est exposé à une source d'énergie dans le proche infrarouge tandis que le treillis chirurgical est en contact avec le tissu, où le matériau absorbant comprend des nanoparticules et est un matériau absorbant dans le proche infrarouge.

2. Treillis chirurgical selon la revendication 1, où le matériau absorbant dans le proche infrarouge présente un pic d'absorbance entre 650 nm et 950 nm.

3. Treillis chirurgical selon la revendication 1, où les nanoparticules sont choisies parmi les nanoparticules métalliques absorbant dans le proche infrarouge, les nanoparticules semi-conductrices absorbant dans le proche infrarouge, les nanoparticules de carbone absorbant dans le proche infrarouge et les colorants absorbant dans le proche infrarouge.

4. Treillis chirurgical selon l'une quelconque des revendications 1-3, où la soudure à tissus est choisie parmi le chitosane, l'albumine, le collagène, la cellulose, l'élastine, les nanopeptides, les dérivés des précédents, et les combinaisons de deux ou plusieurs des précédents.
